# EUROPÄISCHE PATENTSCHRIFT

(11) **EP 2 923 665 B1**
(45) Veröffentlichungstag und Bekanntmachung des Hinweises auf die Patenterteilung: **17.10.2018**
(21) Anmeldenummer: 15153396.5
(22) Anmeldetag: 02.02.2015
(51) Int. Cl.: A61B 17/34, A61M 25/00, A61F 2/24

(54) **Kathetervorrichtung zur minimalinvasiven Implantation eines Gefäßimplantats, insbesondere für die interventionelle, kathetergestützte Aortenklappenimplantation**
Catheter device for the minimally invasive implantation of a vascular implant, in particular for interventional catheter-assisted aortic valve implantation
Dispositif de cathéter destiné à l'implantation mini-invasive d'un implant vasculaire, en particulier pour l'implantation transcathéter de valvule aortique

(30) Priorität: 26.03.2014 US 201461970395 P
(43) Veröffentlichungstag der Anmeldung: 30.09.2015
(73) Patentinhaber: Biotronik AG, 8180 Bülach (CH)
(72) Erfinder: Kaufmann, Ralf, 79540 Lörrach (DE)
(74) Vertreter: Galander, Marcus

(56) Entgegenhaltungen:
- US-A- 5 320 627
- US-A1- 2003 212 411
- US-A1- 2004 093 061
- US-A1- 2005 209 670
- US-A1- 2006 041 302
- US-A1- 2013 204 345

## Beschreibung

Die Erfindung betrifft eine Kathetervorrichtung zur minimalinvasiven Implantation eines Gefäßimplantats, insbesondere für die interventionelle, kathetergestützte Aortenklappenimplantation. Die vorliegende Erfindung wird hauptsächlich am Beispiel einer Aortenklappenimplantation beschrieben, ist jedoch nicht auf diese Anwendung eingeschränkt. Die vorliegende Erfindung ist prinzipiell für jede Kathetervorrichtung geeignet, bei der ein äußerer Katheterschlauch axial in Bezug auf einen inneren Katheterschlauch bewegt wird. Dies kann auch eine Kathetervorrichtung zur Freisetzung eines selbstexpandierenden Stents sein.

Die interventionelle, kathetergestützte Aortenklappenimplantation ist ein Verfahren zur Behandlung von Patienten mit hochgradiger Aortenklappenstenose. Dabei wird die Implantation analog einer Herzkatheteruntersuchung über die Schlagader in der Leiste (transfemoral), einen kleinen Schnitt in der linken Brustkorbseite über die Herzspitze (transapikal) oder über eine obere partielle Stemotomie direkt durch die Körperschlagader (transaortal) des Patienten durchgeführt. Zunächst wird am schlagenden Herzen die verengte, körpereigene Aortenklappe mit einem Ballon ausgedehnt - Ballonvalvuloplastie - und anschließend eine Gefäßprothese mit integrierter biologischer Herzklappe über einen Katheter eingebracht und auf Höhe der erkrankten Aortenklappe zur Implantation entfaltet.

Um diese Funktionalität zu erreichen, weist eine solche Kathetervorrichtung zur Aortenklappenimplantation einen äußeren Katheterschlauch mit einem distalen und einem proximalen Ende und einen mit Radialabstand darin relativ-verschiebbar geführten, inneren Katheterschlauch auf. Durch die Relativbewegung der beiden Katheterschläuche kann eine bestimmte Funktion der Kathetervorrichtung, wie das Freilegen des Ballons zum Aufweiten der natürlichen Herzklappe oder die Implantation der eigentlichen Klappenprothese mittels Freisetzen der Gefäßprothese realisiert werden.

Eine Herzklappenprothese nach dem Stand der Technik besteht wie eingangs erwähnt im Wesentlichen aus einem Grundgerüst und einer darin integrierten, befestigten Herzklappe. Die eigentliche Herzklappe kann dabei aus natürlichen Gewebe (wie beispielsweise Perikardgewebe vom Schwein oder Rind) oder Polymergewebe (Dacron oder ähnliches) bestehen. Möglich sind auch Spenderklappen. Die Herzklappe wird in dem Grundgerüst befestigt, wobei sie zumeist mit dem Grundgerüst vernäht wird. Das Grundgerüst dient der Verankerung der Gefäßprothese an der Stelle der natürlichen Klappe. Das Grundgerüst ist im Wesentlichen ein Stent und kann dabei entweder selbstexpandierend oder mittels Ballon expandierbar ausgeführt werden. Bei einem Kathetersystem der eingangs erwähnten Art wird die Gefäßprothese während der Einführung und Positionierung am Implantationsort vom äußeren Katheterschlauch überdeckt. Für die Implantation wird der äußere Katheterschlauch zurück gezogen. Im Falle eines selbstexpandierenden Grundgerüstes entfällt durch Zurückziehen des Katheterschlauchs die Kraft, die das Grundgerüst auf dem inneren Katheterschlauch in seinem komprimierten Zustand hält. Das Grundgerüst expandiert dadurch und verankert sich an der Stelle der natürlichen Herzklappe. Im Falle eines ballonexpandierbaren Grundgerüsts wird dieses durch Rückziehen des äußeren Katheterschlauches freigelegt und kann expandiert werden.

Problematisch bei der Aortenklappenimplantation ist die Tatsache, dass die Kathetervorrichtung im Aortenbogen stark gekrümmt wird, so dass die Reibung zwischen äußerem und innerem Katheterschlauch problematisch werden kann. Insbesondere kann die durch die Krümmung hervorgerufene Reibungserhöhung dazu führen, dass die Relativverschiebung der Katheterschläuche schwergängig vonstatten geht, so dass die Steuerbarkeit der Katheterfunktionen beeinträchtigt werden kann.

Der Stand der Technik offenbart hinsichtlich dieser Problematik einige Lösungsansätze. So ist es aus der US 2006/0229574 A1 im Zusammenhang mit ineinandersitzenden Katheterlumen bekannt, eine in Kontakt mit anderen Katheterflächen gelangende Oberfläche mit Höckern und Vertiefungen zu versehen. Die Reibung einer so gewellten Oberfläche mit einer danebenliegenden glatten Fläche wird insgesamt reduziert, so dass eine relative Bewegung mehrerer Katheterschläuche zueinander leichtgängig erfolgen kann.

Die EP 1 459 706 A1 offenbart ein Verfahren zum Anbringen eines selbstexpandierenden Stents auf einem Katheter, wobei mindestens eine der beteiligten Komponenten - also der Stent selbst, entsprechende Mittel zur Kompression des Stents, das beteiligte Transferrohr oder der Katheter selbst - zur Reduktion der Reibung bei relativ zueinander beweglichen Bauteilen mit einem Gleitbeschichtung versehen ist, die aus einem biokompatiblen Schmiermittel besteht. Als bevorzugtes Beispiel für ein solches Gleitmittel wird ein Glycerol-haltiger Schmierstoff genannt.

Aus der EP 0 747 073 A1 ist es bekannt, die Außenfläche des inneren Katheterschlauches einer medizinischen Kathetervorrichtung und die Innenflächen deren äußeren Katheterschlauches mit einer Fluidschicht zu versehen, um die Probleme bei einer Reibung von Innen- zu Außen-Katheterschlauch bei einer Relativbewegung zu beheben. Als bekannt werden in diesem Zusammenhang die Verwendung von Materialien mit einem geringen Reibungskoeffizienten, wie beispielsweise PTFE bzw. die Anbringung eines Schmiermittels beispielsweise in Form eines Hydrogels genannt. Darüber hinaus lehrt diese Druckschrift die Aufbringung einer permanenten Fluidschicht in Form eines Heparin-haltigen Hydrogels.

Ein Abgabesystem für eine selbstexpandierende künstliche Herzklappe unter Verwendung einer Rollmembran ist aus der WO 2010/033698 A1 entnehmbar. Dabei wird die Reibung zwischen der Katheterhülle und der zu implantierenden Herzklappe durch den Abrollvorgang der Rollmembran auf mechanischem Wege deutlich reduziert. Eine solche Rollmembran ist allerdings nur am Ende einer Kathetervorrichtung einsetzbar.

Die US 2011/0213303 A1 schließlich offenbart einen Katheter, der eine so bezeichnete "Gänsehaut"-ähnliche Oberflächenstruktur zur drastischen Reduzierung der Reibung der so ausgerüsteten Fläche bewirkt. Die "Gänsehaut"-Struktur wird durch Verwendung eines thermoplastischen Materials in Form von Polyamid und einem Elastomer, wie einem Polyesterblockamid, realisiert, in das mikroskopisch kleine Partikel, wie beispielsweise Glasperlen, oberflächlich eingebettet sind. Der damit erreichbare Reibungskoeffizient wird mit dem von PTFE verglichen.

US 2005/0209670 offenbart ein Verfahren und eine Anordnung zur Einführung und Implantation von Prothesen in ein Körperlumen. Dabei wird ein selbstexpandierender Stent in Verbindung mit einer Angioplastie mit einem Einführsystem für den Stent offenbart, wobei das Einführsystem eine Fixierung für den Stent aufweist, welche ihre Durchmesser adaptieren kann.

US 2005/0093061 offenbart einen Einführkatheter für die Einführung von mehreren, einzelnen Prothesen in ein Körperlumen. Der Katheter umfasst einen Schaft, einen Pusher zur Bewegung der Prothesen relativ zu diesem Schaft und eine Klappenanordnung, um selektiv Prothesen im Schaft zu halten.

US 2003/0212411 offenbart eine Vorrichtung zur Schaffung eines Zugangs in den Spinalkanal eines Patienten. Die Vorrichtung umfasst einen Introducer und eine Schutzhülle, die mit einem Ende am proximalen Ende des Introducer befestigt ist.

US 2013/0204345 offenbart eine Vorrichtung, ein System und ein Verfahren umfassend einen invertierten Schaft. Dieser Schaft ist derart ausgestaltet, dass er das medizinische Gerät abdeckt und teilweise zusammenpresst. Das medizinische Gerät wird an der Behandlungsstelle durch Zurückziehen des Schaftes mittels Umstülpen freigesetzt.

Der Erfindung liegt die Aufgabe zugrunde, eine Kathetervorrichtung zur minimalinvasiven Implantation eines Gefäßimplantats mit einem äußeren Katheterschlauch und einem mit Radialabstand darin relativ-verschiebbar geführten inneren Katheterschlauch so weiterzubilden, dass die Reibung zwischen den beiden Schläuchen ohne Verwendung von Oberflächenstrukturen oder -beschichtungen der benachbarten Oberflächen möglichst gering wird.

Diese Aufgabe wird durch die im Kennzeichnungsteil des Anspruches 1 angegebenen Merkmale gelöst, wonach eine Rollenanordnung im Radialabstandsspalt zwischen innerem und äußerem Katheterschlauch vorgesehen ist. Diese weist über einen oder mehrere begrenzte Längenabschnitte verteilte Rollkörper auf, an denen sich der äußere Katheterschlauch mit seiner Innenfläche und der innere Katheterschlauch mit seiner Außenfläche rollend abstützen.

Die erfindungsgemäße Ausgestaltung hat erkennbar den Vorteil, dass völlig herkömmliche Katheterschläuche zur Ausbildung der Kathetervorrichtung verwendet werden können, wobei die Reibungsminimierung durch mechanische Mittel in Form der Rollenanordnung erfolgt. Es sind also keine möglicherweise Nebeneffekte hervorrufende Beschichtungen oder aufwändige Oberflächenstrukturierungen mehr notwendig.

Mit Hilfe der reibungsverminderten Relativverschiebkarkeit von äußerem und innerem Katheterschlauch zueinander ist eine präzisere Freisetzung des Gefäßimplantates erreichbar.

Die Rollkörper sind hantelförmig ausgebildet und mit ihrer Rollachse quer zur Katheterlängsrichtung ausgerichtet. Die endseitigen hantelscheibenförmigen Rollenabschnitte bilden dabei mit ihrem Umfang die Abrollfläche für den äußeren Katheterschlauch. Der Verbindungsbereich zwischen den hantelscheibenförmigen Rollenabschnitten dient als Abrollfläche für den inneren Katheterschlauch. Durch diese hantelförmige Ausgestaltung wird also eine funktionale Trennung unterschiedlicher Zonen der Rollkörper erreicht, so dass diese jeweils optimal an ihren Verwendungszweck angepasst sein können. Unter einem hantelförmigen Körper wird im Rahmen der Anmeldung ein Körper mit einer Rotationsachse verstanden, an deren beiden Enden jeweils ein rotationssymmetrischer Körper, wie beispielsweise eine Scheibe, angeordnet ist.

Alternativ sind die Rollkörper in der Form eines Diabolos ausgebildet. Unter einem Diabolo wird im Rahmen der Anmeldung eine geometrische Figur verstanden, die aus zwei Kegeln oder Kegelstümpfen oder aus zwei Halbkugeln bzw. Kugelhalbschalen zusammen gesetzt ist. Dabei sind die Kegel oder Kegelstümpfe an der spitzen Seite miteinander verbunden. Die beiden Halbkugeln oder Kugelhalbschalen sind derart miteinander verbunden, dass Kugelschnittfläche bzw. die Öffnungen der Kugelhalbschalen nach außen zeigen. Derart entsteht ein Rotationskörper, der um die Verbindungsachse der Kegel bzw. Halbkugeln rotationssymmetrisch ist. Die diaboloförmigen Rollkörper sind dabei zweckmäßigerweise analog zu den hantelförmigen Rollkörpern dimensioniert und ausgerichtet.

So kann beispielsweise die Abrollfläche der Rollkörper für den inneren Katheterschlauch entsprechend dem Außendurchmesser des inneren Katheterschlauches ausgekehlt sein, so dass der innere Katheterschlauch besonders präzise zwischen den Rollkörpern geführt wird.

In einer weiteren bevorzugten Ausgestaltung der Erfindung weist die Innenfläche des Außenkatheters einen Polygonalen Querschnitt auf. Der polygonale Querschnitt ist dabei zweckmäßigerweise an die Dimension und Zahl der Rollkörper pro Querschnitt angepasst. Werden beispielsweise 3 Rollkörper in einem gegebenen Querschnitt des Katheters angeordnet, weist die Innenseite des Außenkatheters zweckmäßigerweise einen Querschnitt in der Form eines regelmäßigen Sechsecks auf. Auf jeweils 3 Seiten des Sechsecks werden so beispielsweise hantel- oder diaboloförmige Rollkörper angeordnet und bewegen sich geführt über die Außenseite des Innenschlauchs bei einer entsprechenden Relativbewegung zwischen Außenschlauch und Innenschlauch.

Eine herstellungstechnisch und gestalterisch einfache Ausbildung der Rollkörper in Form von Kugeln oder Zylindern ist ebenfalls denkbar.

Die Verteilung der Rollkörper innerhalb der Kathetervorrichtung kann in einer bevorzugten Ausführungsform optimiert werden, indem beispielsweise innerhalb eines Längenabschnittes der Kathetervorrichtung eine Gruppe von Rollkörpern in Umfangsrichtung des Radialabstandsspaltes verteilt vorgesehen ist. Damit wird in diesem Längenabschnitt eine saubere Führung der beiden Katheterschläuche relativ zueinander erreicht. Die Positionierung der Längenabschnitte kann dabei jeweils angepasst an den Einsatzzweck der Kathetervorrichtung erfolgen. Insbesondere ist es gemäß einer bevorzugten Ausführungsform der Kathetervorrichtung vorgesehen, die Rollenanordnung mit einem oder mehreren Längenabschnitten nur in dem Längsbereich der Kathetervorrichtung anzuordnen, der bei der Aortenklappenimplantation im Aortenbogen zu liegen kommt. Damit ist der Katheter optimal an diesen Einsatzzweck angepasst.

Weitere Merkmale, Einzelheiten und Vorteile der Erfindung ergeben sich aus der nachfolgenden Beschreibung eines Ausführungsbeispiels anhand der beigefügten Zeichnungen. Es zeigen:
- Fig. 1: eine schematische Perspektivansicht einer Kathetervorrichtung in implantiertem Zustand,
- Fig. 2: einen teilweise weggebrochenen Detailausschnitt der Kathetervorrichtung in perspektivischer Ansicht, und
- Fig. 3: eine schematische Perspektivansicht einer Kathetervorrichtung nach Implantation der Herzklappenprothese.

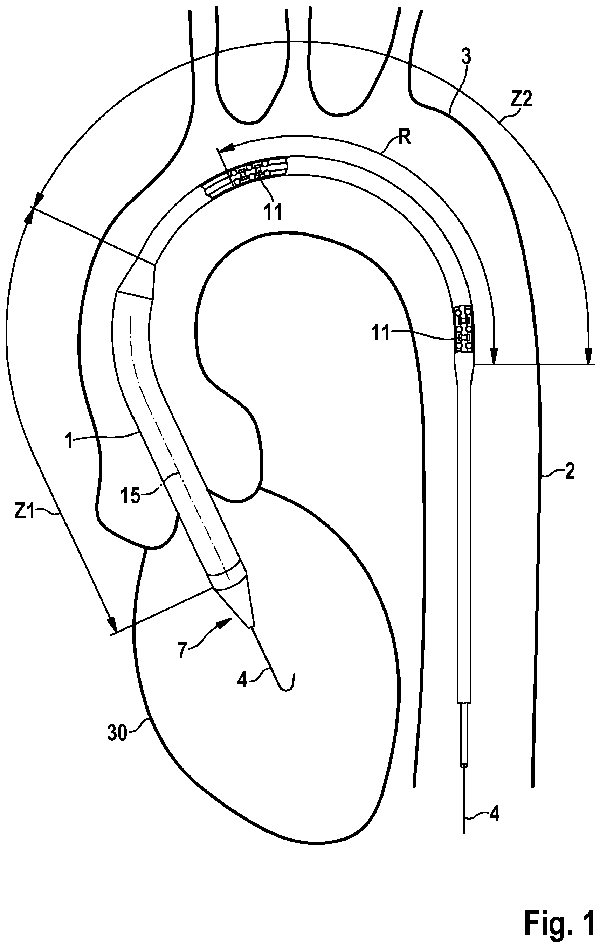

Fig. 1 zeigt eine Kathetervorrichtung 1 für eine interventionelle, kathetergestützte Aortenklappenimplantation, wobei die Kathetervorrichtung 1 in der Aorta 2 mit der Spitze im rechten Ventrikel des Herzens 30 eines Patienten dargestellt ist. Die Kathetervorrichtung 1 weist dabei die stärkste Krümmung im Bereich des Aortenbogens 3 auf. Zur Positionierung der Kathetervorrichtung 1 ist ein Führungsdraht 4 vorgesehen, auf dem dann die Kathetervorrichtung 1 mit einem entsprechenden Lumen 5 in dem inneren Katheterschlauch 6 in die in Fig. 1 gezeigte Position geschoben wird. Am distalen Ende 7 der Kathetervorrichtung 1 ist eine - nicht dargestellte - Klappenprothese montiert, die nach dem Aufweiten der erkrankten Aortenklappe mittels eines Ballons dort platziert wird. Die Klappenprothese nimmt den Abschnitt Z1 der Kathetervorrichtung 1 ein und ist in Fig. 1 vom äußeren Katheterschlauch überdeckt.

Das proximale Ende der Kathetervorrichtung 1 ist in Fig. 1 nicht dargestellt.

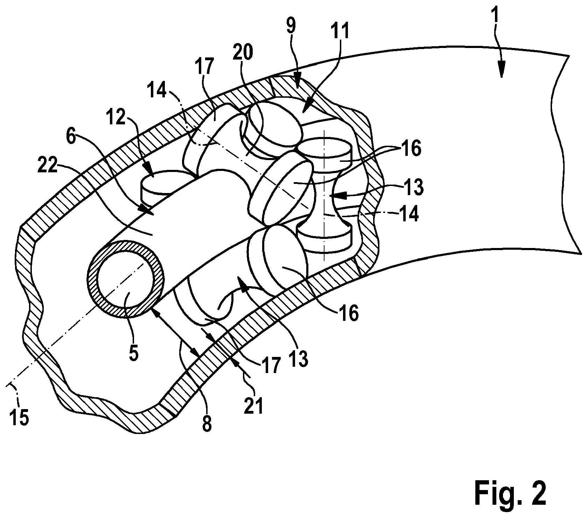

Wie aus Fig. 2 deutlich wird, ist auf dem erwähnten inneren Katheterschlauch 6 mit Radialabstand 8 ein äußerer Katheterschlauch 9 positioniert. Innerer und äußerer Katheterschlauch 6, 9 sind relativ zueinander verschiebbar.

Um insbesondere im Bereich des Aortenbogens 3 mit der großen Krümmung der Kathetervorrichtung 1 eine möglichst reibungsarme Verschiebbarkeit der beiden Katheterschläuche 6, 9 zu gewährleisten, weist die Kathetervorrichtung 1 beispielsweise in den in Fig. 1 angedeuteten Längenabschnitt R jeweils eine in Fig. 2 erkennbare Rollenanordnung 11 im Radialabstandsspalt 12 auf, die jeweils aus einer Mehrzahl von Rollkörpern 13 gebildet ist. Letztere sind jeweils hantelförmig ausgebildet und mit ihrer Rollachse 14 quer zur Katheterlängsachse 15 ausgerichtet. Die endseitigen, handelscheibenförmigen Rollenabschnitte 16 der Rollkörper 13 bilden mit ihrem Umfang die Abrollfläche 17, mit der sie sich an der Innenfläche 21 des äußeren Katheterschlauches 9 rollend abstützen. Die Innenfläche des äußeren Katheterschlauchs 9 weist in dieser Ausgestaltung einen sechseckigen Querschnitt auf, wobei die Seitenlängen des Sechsecks der Länge der hantelförmigen Rollkörper 13 entspricht. Der Verbindungsbereich 18 zwischen den hantelscheibenförmigen Rollenabschnitten 16 ist entsprechend dem Außendurchmesser 19 des inneren Katheterschlauches 16 ausgekehlt, so dass sich der innere Katheterschlauch 6 in diesen Verbindungsbereich 18, der die Abrollfläche 20 für ihn bildet, mit seiner Außenfläche 22 schmiegen und eine saubere Führung des inneren Katheterschlauches 6 relativ zum äußeren Katheterschlauch 9 erzielt werden kann. Dadurch, dass die Rollkörper 13 jeweils gruppenweise im Längenabschnitte R in Umfangsrichtung des Radialabstandsspaltes 12 verteilt angeordnet sind, ist der reibungsvermindernde Effekt der Rollenanordnungen 11 isotrop, d. h. unabhängig von der Krümmungsrichtung der Kathetervorrichtung 1.

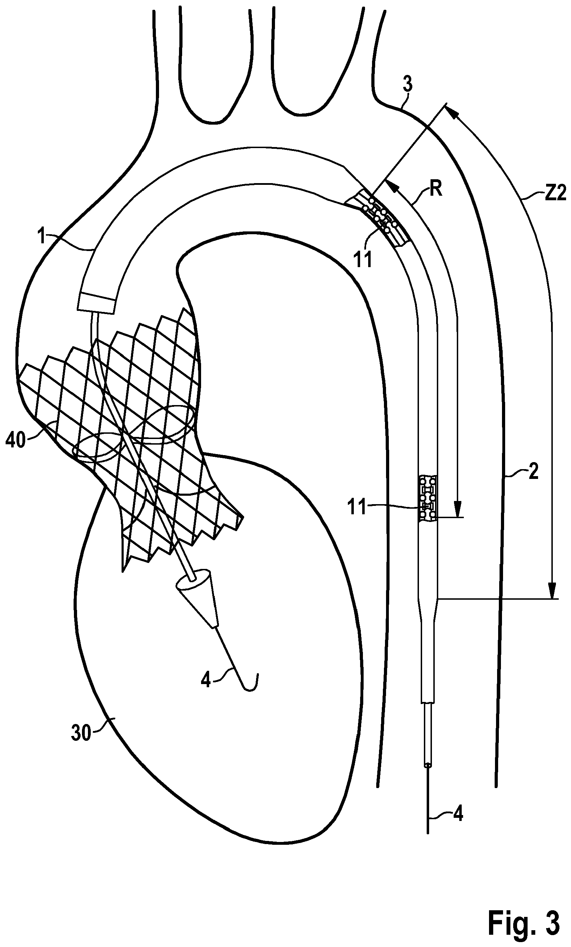

Fig. 3 zeigt die Kathetervorrichtung 1 nach Implantation der Herzklappenprothese 40 im Anulus des Herzens 30. Entsprechend ist der äußere Katheterschlauch 9 hier nahezu vollständig zurück gezogen und die beiden Zonen R und Z2 nach proximal verschoben. Beim Zurückziehen des äußeren Katheterschlauchs 9 führen die Rollkörper 13 wie bereits ausgeführt eine Rollbewegung aus. Dabei rollen die inneren Teile der Rollkörper 13 mit ihrer Abrollfläche 20 auf dem inneren Katheterschlauch 6 während die äußeren scheibenförmigen Abschnitte 16 mit ihrer Abrollfläche 17 auf dem äußeren Katheterschlauch 9 rollen. Aufgrund der unterschiedlichen Durchmessers wird entsprechend die Zone Z2 des äußeren Katheterschlauchs 9 weiter nach proximal zurück verschoben als die Rollzone R.

## Patentansprüche

1. Kathetervorrichtung zur minimalinvasiven Implantation eines Gefäßimplantats, insbesondere für die interventionelle, kathetergestützte Aortenklappenimplantation, umfassend
- einen äußeren Katheterschlauch (9) mit einem distalen (7) und einem proximalen Ende, und
- einen mit Radialabstand (8) darin relativ-verschiebbar geführten inneren Katheterschlauch (6),
- eine Rollenanordnung (11) im Radialabstandsspalt (12) zwischen innerem und äußerem Katheterschlauch (6, 9),
**gekennzeichnet dadurch, dass** die Rollenanordnung über einen oder mehrere begrenzte
Längenabschnitte (10.1, 10.2) der Kathetervorrichtung verteilte Rollkörper (13) aufweist, an denen sich der äußere Katheterschlauch (9) mit seiner Innenfläche (21) und der innere Katheterschlauch (6) mit seiner Außenfläche (22) rollend abstützen, wobei die Rollkörper (13) hantelförmig oder diaboloförmig ausgebildet und mit ihrer Rollachse (14) quer zur Katheterlängsachse (15) ausgerichtet sind, wobei die endseitigen hantelscheibenförmigen Rollenabschnitte (16) mit ihrem Umfang die Abrollfläche (17) für den äußeren Katheterschlauch (9) und der Verbindungsbereich (18) zwischen den hantelscheibenförmigen Rollenabschnitten (16) die Abrollfläche (20) für den inneren Katheterschlauch (6) bilden.

2. Kathetervorrichtung nach Anspruch 1, **dadurch gekennzeichnet, dass** die Abrollfläche (20) der Rollkörper (13) für den inneren Katheterschlauch (6) entsprechend dem Außendurchmesser (19) des inneren Katheterschlauches (6) ausgekehlt ist.

3. Kathetervorrichtung nach Anspruch 1 oder 2, **dadurch gekennzeichnet, dass** die Innenseite des äußeren Katheterschlauchs einen polygonalen, bevorzugt einen sechseckigen, Querschnitt aufweist.

4. Kathetervorrichtung nach einem der vorgenannten Ansprüche, **dadurch gekennzeichnet, dass** innerhalb eines Längenabschnittes (10.1, 10.2) der Kathetervorrichtung (1) eine Gruppe von Rollkörpern (13) in Umfangsrichtung des Radialabstandsspaltes (12) verteilt angeordnet ist.

5. Kathetervorrichtung nach einem der vorgenannten Ansprüche für die interventionelle, kathetergestützte Aortenklappenimplantation, **dadurch gekennzeichnet, dass** die Rollenanordnung (11) mit einem oder mehreren Längenabschnitten (10.1, 10.2) nur in dem Längsbereich der Kathetervorrichtung (1) angeordnet ist, der bei der Aortenklappenimplantation im Aortenbogen (3) zu liegen kommt.

## Claims

1. A catheter device for the minimally invasive implantation of a vascular implant, in particular for interventional catheter-assisted aortic valve implantation, comprising
- an outer catheter tube (9) having a distal end (7) and a proximal end, and
- an inner catheter tube (6) guided therein at a radial distance (8) in a manner displaceable relative thereto,
- a roller arrangement (11) in the radial gap (12) between the inner and outer catheter tube (6, 9),
**characterised in that** said roller arrangement has rolling elements (13) distributed over one or more delimited length portions (10.1, 10.2) of the catheter device, the outer catheter tube (9) being supported in a rolling manner via its inner face (21) on said rolling elements and the inner catheter tube (6) being supported in a rolling manner via its outer face (22) on said rolling elements, wherein the rolling elements (13) are barbell-shaped or diabolo-shaped and are oriented with their rolling axis (14) transverse to the longitudinal axis (15) of the catheter, wherein the periphery of the weight-plate-shaped roller portions (16) arranged at the end forms the rolling surface (17) for the outer catheter tube (9) and the connection region (18) between the weight-plate-shaped roller portions (16) forms the rolling surface (20) for the inner catheter tube (6).

2. The catheter device according to claim 1, **characterised in that** the rolling surface (20) of the rolling elements (13) for the inner catheter tube (6) is grooved in accordance with the outer diameter (19) of the inner catheter tube (6).

3. The catheter device according to claim 1 or 2, **characterised in that** the inner face of the outer catheter tube has a polygonal cross section, preferably a hexagonal cross section.

4. The catheter device according to any one of the preceding claims, **characterised in that** a group of rolling elements (13) is distributed in the peripheral direction of the radial gap (12) within a length portion (10.1, 10.2) of the catheter device (1).

5. The catheter device according to any one of the preceding claims for interventional catheter-assisted aortic valve implantation, **characterised in that** the roller arrangement (11) with one or more length portions (10.1, 10.2) is arranged only in the longitudinal region of the catheter device (1) that comes to rest in the aortic arch (3) during aortic valve implantation.

## Revendications

1. Dispositif de cathéter pour une implantation mini-invasive d'un implant vasculaire, notamment, pour l'implantation interventionnelle d'une valve aortique assurée par cathéter, comprenant
- un tube de cathéter extérieur (9) avec une extrémité distale (7) et une extrémité proximale, et
- un tube de cathéter intérieur (6) mené à l'intérieur en pouvant être déplacé relativement avec un espacement radial (8),
- un agencement de roulement (11) dans la fente de l'espacement radial (12) entre les tubes de cathéter intérieur et extérieur (6, 9),
**caractérisé en ce que** l'agencement de roulement présente des corps roulants (13) distribués sur un ou plusieurs segments longitudinaux (10.1, 10.2) du dispositif de cathéter, sur lesquels s'appuient tout en roulant le tube de cathéter extérieur (9) avec sa surface intérieure (21) et le tube de cathéter intérieur (6) avec sa surface extérieure (22), où les corps roulants (13) sont conçus en forme d'haltères ou en forme de diabolos et sont orientés perpendiculairement par rapport à l'axe longitudinal de cathéter (15) avec leur axe de rotation (14), où les segments de rouleau (16) en forme de disques d'haltères du côté des extrémités forment avec leur envergure la surface de déroulement (17) pour le tube de cathéter extérieur (9) et la région de liaison (18) entre les segments de rouleau (16) en forme de disques d'haltères forme la surface de déroulement (20) pour le tube de cathéter intérieur (6).

2. Dispositif de cathéter selon la revendication 1, **caractérisé en ce que** la surface de déroulement (20) des corps de rouleaux (13) pour le tube de cathéter intérieur (6) est évasée pour correspondre au diamètre extérieur (19) du tube de cathéter intérieur (6).

3. Dispositif de cathéter selon la revendication 1 ou 2, **caractérisé en ce que** le côté intérieur du tube de cathéter extérieur présente une section transversale polygonale, de préférence une section transversale hexagonale.

4. Dispositif de cathéter selon l'une des revendications précitées, **caractérisé en ce qu'**à l'intérieur d'un segment longitudinal (10.1, 10.2) du dispositif de cathéter (1) un groupe de corps de rouleaux (13) est disposé, distribué dans la direction circonférentielle de la fente de l'espacement radial (12).

5. Dispositif de cathéter selon l'une des revendications précitées pour l'implantation interventionnelle d'une valve aortique assurée par un cathéter, **caractérisé en ce que** l'ensemble de rouleaux (11) est disposé uniquement dans la zone longitudinale du dispositif de cathéter (1) avec un ou plusieurs segments longitudinaux (10.1, 10.2), qui devient adjacente lors de l'implantation de la valve aortique dans l'arc aortique (3).
